(19) Europäisches Patentamt
European Patent Office
Office européen des brevets

(11) Veröffentlichungsnummer: **0 242 851**
**B1**

(12) # EUROPÄISCHE PATENTSCHRIFT

(45) Veröffentlichungstag der Patentschrift: **13.06.90**

(51) Int. Cl.⁵: **C07C 233/01**, A61K 31/165

(21) Anmeldenummer: **87105846.7**

(22) Anmeldetag: **21.04.87**

(54) **N-(2'-Aminophenyl)-benzamid-Derivate, Verfahren zu deren Herstellung und deren Verwendung bei der Bekämpfung neoplastischer Erkrankungen.**

(30) Priorität: **22.04.86 DE 3613571**
**26.07.86 DE 3625359**

(43) Veröffentlichungstag der Anmeldung:
**28.10.87 Patentblatt 87/44**

(45) Bekanntmachung des Hinweises auf die Patenterteilung:
**13.06.90 Patentblatt 90/24**

(84) Benannte Vertragsstaaten:
**AT BE CH DE ES FR GB GR IT LI LU NL SE**

(56) Entgegenhaltungen:
**EP-A- 0 116 967**
**DE-A- 1 643 264**
**FR-M- 5 720**
**GB-A- 2 135 998**

**CHEMICAL ABSTRACTS, Band 105, Nr. 7, 18. August 1986, Seite 574, Zusammenfassungsnr. 60164c, Columbus, Ohio, US; V.K. SHCHEL'TSYN et al.: "Cyclodehydration and hydrolysis of o-aminoanilides. II. 4-Substituted 2'-aminobenzanilides"**
**CHEMICAL ABSTRACTS, Band 103, Nr. 19, 11. November 1985, Seite 39, Zusammenfassungsnr. 153473b, Columbus, Ohio, US; P. LELIEVELD et al.: "Effectiveness of p-aminobenzoyl-O-phenylenediamine (Goe 1734)**

(73) Patentinhaber: **GÖDECKE AKTIENGESELLSCHAFT, Salzufer 16, D-1000 Berlin 10(DE)**

(72) Erfinder: **Weiershausen, Ute, Bundesstrasse 70, D-7803 Gundelfingen(DE)**
Erfinder: **Satzinger, Gerhard, Dr., Im Mattenbühl 7, D-7809 Denzlingen(DE)**
Erfinder: **Vollmer, Karl-Otto, Dr., Sulzburgerstrasse 56, D-7800 Freiburg(DE)**
Erfinder: **Herrmann, Wolfgang, Dr., Zum Baumgarten 13, D-7802 Merzhausen(DE)**

(56) Entgegenhaltungen: (Fortsetzung)
**against mouse, rat, and human tumor cells"**
**CHEMICAL ABSTRACTS, Band 86, Nr. 23, 6. Juni 1977, Seite 515, Zusammenfassungsnr. 171034u, Columbus, Ohio, US; A.M. ISLAM et al.: "4-Hydroxybenzanilide derivatives of expected biological activity"**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 242 851 B1

ACTORUM AG

**Beschreibung**

Die vorliegende Erfindung befaßt sich mit neuen N-(2'-Aminophenyl)-benzamid-Derivaten, Verfahren zu deren Herstellung, diese enthaltende Arzneimittel und deren Verwendung zur Bekämpfung neoplastischer Erkrankungen.

In der DE-OS 3 305 755 werden Verbindungen der allgemeinen Formel I

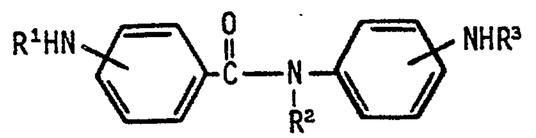

I

in welcher $R^1$, $R^2$ und $R^3$, die gleich oder verschieden sein können, ein Wasserstoffatom oder eine Methylgruppe bedeuten,
als wirksam bei der Bekämpfung von malignen, proliferativen und autoimmunen Erkrankungen beschrieben. Besonders wirksam sind das 4-Amino-N-(2'-aminophenyl)-benzamid und seine N-Monomethylderivate.

Überraschenderweise wurde nun gefunden, daß die zunächst pharmakologisch für essentiell gehaltene, basische p-Amino-funktion nicht nur nicht verantwortlich für die therapeutische Wirksamkeit der erfindungsgemäßen Substanzen ist, sondern ihre Abwesenheit oder ihre chemische Veränderung in neutral reagierende Gruppen durch Substitution bzw. ihr Ersatz durch nicht-basische Reste zu wirksamen Verbindungen mit überlegener Verträglichkeit führen.

Gegenstand der Erfindung sind somit neue N-2(Aminophenyl)-benzamid-Derivate zur Therapie maligner, proliferativer und autoimmuner Erkrankungen der allgemeinen Formel II

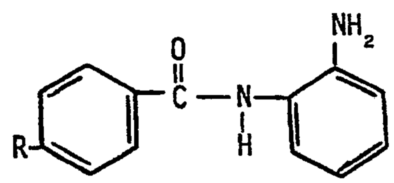

II

worin
R eine unsubstituierte oder mit Hydroxyl substituierte $C_1$ bis $C_4$ Acylaminogruppe bedeutet.

Bevorzugt sind dabei Verbindungen wie
1) 4-Acetamino-N-(2'-aminophenyl)-benzamid
2) 4-Isobutyrylamino-N-(2'-aminophenyl)-benzamid
3) 4-Formylamino-N-(2'-aminophenyl)-benzamid
4) 4-(ß-Hydroxypropionyl)amino-N-(2'-aminophenyl)-benzamid
5) 4-Glycoloylamino-N-(2'-aminophenyl)-benzamid

Die 4-Acylamino-Verbindungen 1) bis 5) sowie die anderen oben aufgeführten N-(2'-Aminophenyl)-benzamid-Derivate der allgemeinen Formel II sind neu. Bekannt ist jedoch eine Verbindung mit R = H (Beilstein 13, HW S. 20), ohne daß deren pharmakologischen Wirkung bisher beschrieben wurde.

Ein weiterer Gegenstand der Erfindung ist daher die Verwendung von Verbindungen der allgemeinen Formel II, wobei jedoch R zusätzlich auch Wasserstoff sein kann, bei der Bekämpfung neoplastischer Erkrankungen.

Die Verbindungen der allgemeinen Formel II können hergestellt werden, indem man Verbindungen der allgemeinen Formel III a und b:

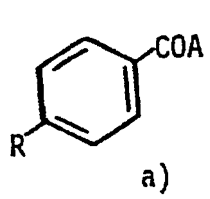

a)

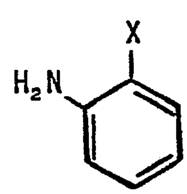

b)

(III a) + b)),

in welcher A eine reaktive Säuregruppe darstellt, R die obengenannte Bedeutung hat und X eine mit einer Schutzgruppe versehene Aminogruppe oder eine Nitrogruppe bedeutet,
in an sich bekannter Weise miteinander umsetzt, die so erhaltenen Verbindungen der allgemeinen Formel IV,

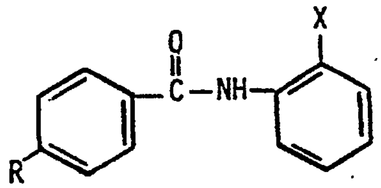

(IV),

in welcher X und R die obengenannte Bedeutung haben reduziert oder durch Abspaltung der Schutzgruppe in die entsprechenden Verbindungen der Fomel II überführt.

Die Umsetzung der Verbindungen gemäß Formel IIIa mit Verbindungen der Formel IIIb erfolgt in an sich bekannter Weise. Als reaktive Säuregruppen A dienen vor allem die Säurehalogenide, -anhydride oder -imidazolide oder Estergruppen, die eine Umsetzung mit der Aminogruppe erlauben. A bedeutet somit bevorzugt Halogen, Imidazolyl-, Acyl- oder niedere Alkoxyreste.

Als Schutzgruppen für X dienen die in der Peptidchemie üblichen Reste, wie z.B. der Benzyl- oder der Carbobenzoxy-Rest.

Die Reduktion kann unter Vewendung geeigneter Katalysatoren (Platin oder Palladium) mit Wasserstoff so durchgeführt werden, daß einerseits die freien Nitrogruppen zu primären Aminogruppen reduziert werden und andererseits die an den Aminogruppen befindlichen Schutzgruppen hydrogenolytisch abgespalten werden.

Die erfindungsgemäßen Wirkstoffe werden vorteilhaft in Form eines pharmazeutischen Präparats verabreicht, welches die Wirkstoffe in freier Form oder in Form einer Mischung mit einem z.B. für die topische, enterale (z.B. orale oder rectale) oder parenterale (intramuskuläre oder intravenöse) Applikation geeigneten pharmazeutischen organischen oder anorganischen festen oder flüssigen Trägermaterial enthält. Für die Bildung derselben kommen solche Stoffe in Frage, die mit den neuen Verbindungen nicht reagieren, wie z.B. Gelatine, Lactose, Stärke, Stearylalkohol, Magnesiumstearat, Talk, pflanzliche Öle, Benzylalkohole, Prophylenglykole, Vaseline oder andere Arzneimittelträger.

Die pharmazeutischen Präparate können z.B. als Tabletten, Dragées, Kapseln, Suppositorien, Salben, Cremes oder in flüssiger Form als Suspensionen oder Emulsionen vorliegen. Gegebenenfalls sind sie sterilisiert und/oder enthalten Hilfsstoffe, wie Konservierungs-, Stabilisierungs-, Netz- oder Emulgiermittel, Lösungsvermittler oder Salze zur Veränderung des osmotischen Druckes oder Puffer. Sie können auch weitere Wirkstoffe enthalten.

Die anzuwendende Dosis hängt von der Art des zu therapierenden Krankheitsgeschehens und von individuellen Faktoren ab.

Im allgemeinen werden Dosen von 10 bis 300 mg, insbesondere 20 bis 50 mg verabreicht. In besonderen Fällen kann die Einzeldosis auch höher liegen.

<u>Beispiel 1</u>

<u>4-Acetamino-N-(2'-aminophenyl)-benzamid</u>

30 g (0.1 mol) N-(2'-Nitrophenyl)-4-acetylaminobenzamid werden unter Standardbedingungen in THF mit Pd/C (10%) hydriert. Nach Entfernen des Katalysators wird das Filtrat auf ca. 1/4 eingeengt und der ausgefallene Niederschlag abgesaugt. Eventuell kann das Produkt aus Methanol/THF (1:1) umkristallisiert werden.
Ausbeute: 18.6 g (69% d. Th.)
Schmp.: 243.7°C

Das als Ausgangsprodukt verwendete N-(2'-Nitrophenyl)-4-acetylaminobenzamid wird wie folgt hergestellt:

41.3 g (0.33 mol) Oxalylchlorid werden bei 0–5°C unter Feuchtigkeitsausschluss zu einer Lösung von 60.3 g (0.83 mol) DMF in 1.5 l trockenem Essigester getropft. Nach 30 Minuten Rühren bei dieser Temperatur fügt man 44.8 g (0.25 mol) 4-Acetamidobenzoesäure zusammen mit 27.7 g (0.35 mol) Pyridin hinzu und entfernt das Eisbad.

Nach 3 Stunden Rühren bei Raumtemperatur versetzt man die Reaktionsmischung mit einer Lösung aus 38 g (0.28 mol) o-Nitroanilin und 27.7 g (0.35 mol) Pyridin in 30 ml trockenem Essigester.

Nach 15 Stunden Rühren bei Raumtemperatur versetzt man mit 500 ml 1N-NaOH, trennt die Phasen und schüttelt die wäßrige Phase noch dreimal mit je 150 ml Essigester aus. Die vereinigten organischen Phasen werden mit Wasser neutral gewaschen, über Natriumsulfat getrocknet und auf ca. 1/3 eingeengt. Der ausgefallene Niederschlag wird abgesaugt und entweder durch Umkristallisation oder Säulenchromatographie gereinigt.
Ausbeute: 15 g (20% d. Th.)
Schmp.: 205.8°C

Beispiel 2

4-(ß-Hydroxypropanoyl)amino-N-(2′-aminophenyl)-benzamid

2 g (4.76 mmol) N-(2′-Nitrophenyl)-4-(3-Benzyloxypropanoylamino)-benzamid werden in 300 ml Ethanol gelöst und unter Zusatz von 1 g Pd/C (5%) bei 80% für 6 Stunden im Autoklaven hydriert. Nach der Filtration wird das Lösungsmittel entfernt und der Rückstand durch Säulenchromatographie (Kieselgel, Methylenchlorid/Methanol 9:1) gereinigt.
Ausbeute: 0–8 g (56%); Schmp.: 198.7°C

Die als Ausgangsprodukte verwendeten Verbindungen werden wie folgt hergestellt:

4-(3-Benzyloxypropanoylamino)-benzoesäure

8.09 g (59 mmol) p-Aminobenzoesäure werden zusammen mit 4.98 g (63 mmol) Pyridin in 100 ml Dioxan gelöst und bei 15°C mit 11.7 g (59 mmol) 3-Benzyloxypropionsäurechlorid (J.C.S. Perkin I, 1976, 2235) in 2 ml Dioxan versetzt.

Nach 3 Stunden Rühren bei Raumtemperatur versetzt man die Reaktionsmischung mir 300 ml Wasser. Der ausgefallene Niederschlag wird abgesaugt, mit Wasser gewaschen und getrocknet. Das Produkt wird ohne weitere Reinigung in die nächste Stufe eingesetzt.
Ausbeute: 15.7 g (89%) Schmp.: 162–164° Sintern > 200°C Zersetzung

N-(2′-Nitrophenyl)-4-(3-Benzyloxypropanoylamino)benzamid wird in Analogie zu Beispiel 1 hergestellt.
Eingesetzte Mengen:
12 g (40.1 mmol) 4-(3-Benzyloxypropanoylamino)-benzoesäure
6.6 g (0.13 mmol) Oxalylchlorid
9.7 g (0.13 mol) Dimethylformamid
2×4.5 g Pyridin
6.1 g (44.1 mmol) 0-Nitroanilin
280 ml getrockneter Essigester

Das Rohprodukt wurde durch Säulenchromatographie (Kieselgel, Essigester/n-Hexan 1:5, 1:1) gereinigt.
Ausbeute: 3.5 g (21%) Schmp.: 140 °C

Beispiel 3

4-Isobutyrylamino-N-(2′-aminophenyl)-benzamid

13.1 g (40 mmol) N-(2′-nitrophenyl)-4-isobutyrylaminobenzamid werden mit Pd/C (10%) in 400 ml Tetrahydrofuran unter Standardbedingungen hydriert. Nach Einengen des Lösungsmittels auf 120 ml werden die ausgefallenen Kristalle abgesaugt.
Ausbeute: 10.2 g (86%), Schmp.: 247.6°C

Die als Ausgangsprodukte verwendeten Verbindungen werden wie folgt hergestellt:

4-Isobutyrylaminobenzoesäure

50 g (0.36 mol) p-Aminobenzoesäure und 30 g (0.38 mol) Pyridin werden in 600 ml Dioxan gelöst und bei 15°C unter Feuchtigkeitsausschluß tropfenweise mit 40.5 g (0.38 mol) Isobuttersäurechlorid versetzt. Nach 2 Stunden Rühren bei Raumtemperatur werden 500 ml Wasser unter kräftigem Rühren zugegeben. Der ausgefallene Niederschlag wird abgesaugt, mit Wasser gewaschen, getrocknet und aus Diisopropylether/ Essigester (3:4) umkristallisiert. Ausbeute 22 g (30%), Schmp.: 241°C

N-, (2′-Nitrophenyl)-4-isobutyrylaminobenzamid:

N-(2′-Nitrophenyl)-4-isobutyrylaminobenzamid wurde unter den Reaktionsbedingungen gemäß Beispiel 5 hergestellt.

Nach Zugabe von Pyridin/0-Nitroanilin wurde die Reaktionslösung 15 Stunden bei Raumtemperatur und 5 Stunden bei Siedetemperatur gerührt.
Eingesetzte Mengen:
54.6 g (74.68 mmol) Dimethylformamid
1.5 l trockener Essigester
37.3 g (29.42 mmol) Oxalylchlorid
47 g (22.63 mmol) 4-Isobutyrylaminobenzoesäure
25.6 g (32.36 mmol) Pyridin
34.4 g (24.89 mmol) 0-Nitroanilin
25.6 g (32.36 mmol) Pyridin

Die Substanz wurde aus Essigester umkristallisiert.
Ausbeute: 13.3 g (18%), Schmp.: 237.6°C

Beispiel 4

4-Glycoloylamino-N-(2'-aminophenyl)-benzamid

6.08 g (15 mmol) N-(2'-Nitrophenyl)-4-Benzyloxyacetaminobenzamid werden in 400 ml Ethanol und 200 ml Tetrahydrofuran gelöst und 19 Stunden bei 80°C und 50 bar-$H_2$ Druck mit Pd/C 5% (3 g) hydriert. Der Autoklaveninhalt wird heiß filtriert und das farblose Filtrat zur Trockne eingeengt. Der kristalline Rückstand wird aus 800 ml Methanol umkristallisiert.
Ausbeute: 2.6 g (63.2%) Schmp.: 221–223°C

Die als Ausgangsprodukte verwendeten Verbindungen wurden wie folgt hergestellt:
4-Benzyloxyacetaminobenzoesäure

35.7 G (0.26 mol) p-Aminobenzosesäure werden zusammen mit 23.7 g (0.30 mol) Pyridin in 420 ml Dioxan gelöst und bei 15°C mit 51.7 g (0.28 mol) Benzyloxyessigsäurechlorid (Heterocyclic Chem. 15, 601, 1978) tropfenweise versetzt.

Nach 2 Stunden Rühren bei Raumtemperatur versetzt man die Reaktionsmischung mit 300 ml Wasser. Der ausgefallene Niederschlag wird abgesaugt, mit Wasser gewaschen und getrocknet. Das Produkt wird ohne weitere Reinigung in die nächste Stufe eingesetzt.
Ausbeute: 73 g (98.3%) Schmp.: 178–179°C

N-(2-Nitrophenyl)-4-Benzyloxyacetaminobenzamid

28.5 g (0.39 mol) trockenes Dimethylformamid in 910 ml trockenem Essigester werden unter Stickstoffatmosphäre bei 2°C mit 21.5 g (0.17 mol) Oxalylchlorid tropfenweise versetzt. Nach 30 Minuten Rühren bei 2–5°C gibt man eine Suspension aus 37.2 g (0.13 mol) 4-Benzyloxyacetataminobenzoesäure und 14.4 g (0.18 mol) Pyridin in 65 ml Essigester zu und entfernt das Eisbad.

Nach 2 1/2 Stunden Rühren bei Raumtemperatur versetzt man die Reaktionsmischung mit einer Lösung von 19.8 g (0.14 mol) 0-Nitroanilin und 14.4 g (0.18 mol) Pyridin in 65 ml Essigester.

Nach 1 Stunde Rühren bei Raumtemperatur wird 3 Stunden zum Sieden erhitzt. Nach dem Abkühlen wird mit 500 ml 1N-NaOH versetzt und die Phasen getrennt. Die wäßrige Phase wird noch 2× mit Essigester ausgeschüttelt. Die vereinigten organischen Phasen werden mit Wasser neutral gewaschen und über Natriumsulfat getrocknet.

Das Lösungsmittel wird auf 150 ml eingeengt und die ausgefallenen Kristalle werden abgesaugt und aus 700 ml Ethanol umkristallisiert.
Ausbeute: 13 g (24.7%) Schmp.: 128–130°C

Beispiel 5

4-Formylamino-N-(2'-aminophenyl)benzamid

N-(2'-Aminophenyl)-formylamidobenzamid

1.92 g (67.3 mmol) N-(2'-Nitrophenyl)-4-formylamidobenzamid werden mit Pd/C (10%) in 500 ml Tetrahydrofuran unter Standardbedingungen hydriert. Nach Entfernen des Katalysators wird das Lösungsmittel im Vakuum entfernt und der kristalline Rückstand aus Tetrahydrofuran/-Diisopropylether (1/1) umkristallisiert.
Ausbeute: 1.5 g (88.5%) Schmp.: 196.7°C (Z)

Die als Ausgangsprodukt verwendete Verbindung wurde wie folgt hergestellt:
N-(2'-Nitrophenyl)-4-formylamidobenzamid

7.9 g (0.11 mol) Dimethylformamid und 80 ml trockener Essigester werden unter Stickstoffatmosphäre bei 0–5°C mit 5.9 g (46.8 mmol) Oxalylchlorid tropfenweise versetzt. Nach 30 Minuten Rühren bei dieser Temperatur wird die Reaktionsmischung mit 5.95 g (36 mmol) Formyl-4-amidobenzoesäure (Chem. Ber. 23, 3625, 1890; Beilsteins Handbuch der Organischen Chemie, Springer Verlag, 14, 432, 1931) und 4.3 g (54 mmol) Pyridin versetzt und das Eisbad entfernt. Nach 3 Stunden Rühren bei Raumtemperatur wird die Lösung mit 5.47 g (39.6 mmol) 0-Nitroanilin in 15 ml trockenem Essigster versetzt und 15 Stunden bei Raumtemperatur gerührt. Anschließend wird die Lösung mit Ammoniak alkalisiert, mit Wasser gewaschen und über Natriumsulfat getrocknet. Nach Entfernen des Lösungsmittels bleibt ein kristalliner Rückstand der aus Essigester umkristallisiert wird.
Ausbeute: 1.5 g (14.6%) Schmp.: 237.6°C

Biologisches Verhalten im Vergleichsversuch

4-Acetamino-N-(2'-aminophenyl)-benzamid wurde als Beispiel der erfindungsgemäßen Wirkstoffe im Vergleich zum Standard 4-Amino-N-(2'-aminophenyl)-benzamid, für welchen in vitro und in vivo ausgeprägte Inhibition verschiedener experimenteller Tumoren bekannt ist, auf Antitumorwirkung geprüft.

Die Testung wurde in vitro im «Colorimetric Cytotoxity Assay» an L1210 und Mammaadenocarcinom

16C Tumorzellen durchgeführt. Substanzen mit $IC_{50}$ Werten ≤ 250 mg/ml werden in diesem Testsystem als cytostatisch aktiv beurteilt.

Es konnte gezeigt werden (s.Tab. 1), daß der erfindungsgemäße Wirkstoff 4-Acetamino-N-(2'aminophenyl)-benzamid.

1. ausgeprägt cytostatisch wirkt
2. eine mit dem Vergleichsstandard etwa gleiche cytostatische Aktivität aufweist.

Tab. 1

| L 1210 Colorimetric cytotoxicity assay | |
|---|---|
| Test Substanz | $IC_{50}$ µg/ml |
| 4-Acetamino-N-(2'-aminophenyl)-benzamid | 3.75 |
| 4-Amino-N-(2'-aminophenyl)-benzamid | 2.78 |
| 4-Methylamino-N-(2'-aminophenyl)-benzamid | 4.04 |
| **Mamma Adenocarcinom 16c cytotoxicity assay** | |
| Test Substanz | $IC_{50}$ µg/ml |
| 4-Acetamino-N-(2'-aminophenyl)-benzamid | 0.73 |
| 4-Amino-N-(2'-aminophenyl)-benzamid | 0.69 |
| 4-Isobutyrylamino-N-(2'-aminophenyl)-benzamid | 0.32 |
| 4-Formylamino-N-(2'-aminophenyl)-benzamid | 0.40 |
| 4-(β-hydroxypropionyl)amino-N-(2'-aminophenyl)-benzamid | 0.77 |
| 4-Glycoloylamino-N-(2'-aminophenyl)-benzamid | 0.62 |

Überraschenderweise wurde gefunden, daß der erfindungsgemäße Wirkstoff 4-Acetamino-N-(2'-aminophenyl)-benzamid bei akuter intragastraler Verabreichung beträchtlich weniger toxisch ist als der Vergleichsstandard. Dies wurde im orientierenden Versuch (n = 4) an männlichen Mäusen zur Ermittlung der $LD_{50}$ bei 7 Tage-Beobachtung nachgewiesen (s.Tab. 2)

Tab. 2

| Akute intragastrale Toxizität, Maus ♂ | |
|---|---|
| Testsubstanz | $LD_{50}$ mg/kg |
| 4-Acetamino-N-(2'-aminophenyl)-benzamid | 1600 |
| 4-Amino-N-(2'-aminophenyl)-benzamid | 625 |

In pharmakokinetischen Versuchen an Ratten konnte eine verminderte Resorption als mögliche Ursache der wesentlich verbesserten akuten Verträglichkeit des erfindungsgemäßen Wirkstoffs im Vergleich zum Standard ausgeschlossen werden: Die intestinale Resorption der Substanz erfolgt rasch, vollständig und dosislinear.

Die Bioverfügbarkeit des erfindungsgemäßen Wirkstoffs ist 100%: Es wurde kein statistisch signifikanter Unterschied der AUCs nach Gabe von 10 mg/kg i.v. und i.g. bei Ratten gefunden (s.Abb. 1)

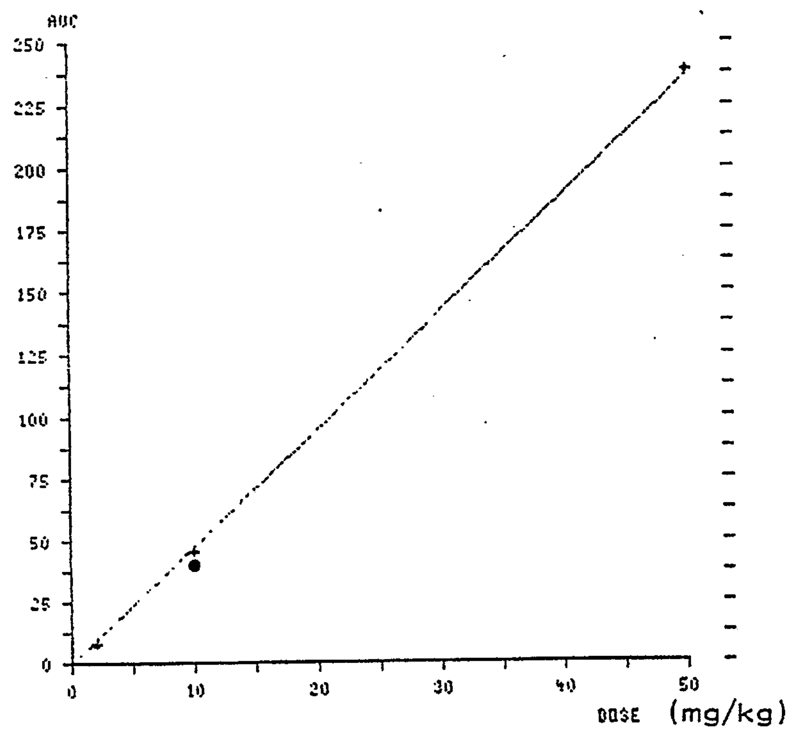


Abb.1: AUC, Ratte, nach Verabreichung von 4-Acetamino-N-(2'-amino-phenyl)-benzamid.
* i.g. Verabreichung (2 mg/kg; 10 mg/kg; 50 mg/kg)
● i.v. Verabreichung (10 mg/kg)

Neben seiner besseren Verträglichkeit zeichnet sich der erfindungsgemäße Wirkstoff gegenüber dem Vergleichsstandard auch durch eine längere Halbwertzeit aus (s. Abb. 2, 3), wodurch es möglich ist, cytostatische Wirkspiegel über längere Zeiträume aufrechtzuerhalten.

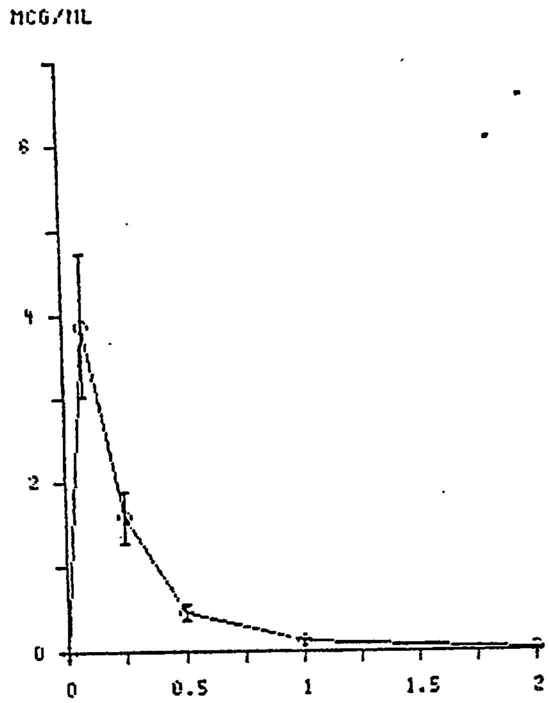


Abb. 2: Mittlerer Plasmaspiegel von 4-Amino-N-(2'-amino-phenyl)-benzamid bei Ratten nach einmaliger Verabreichung von 10 mg/kg i.g. (n = 4).
Halbwertzeit (t1/2) ~ 15 Minuten.

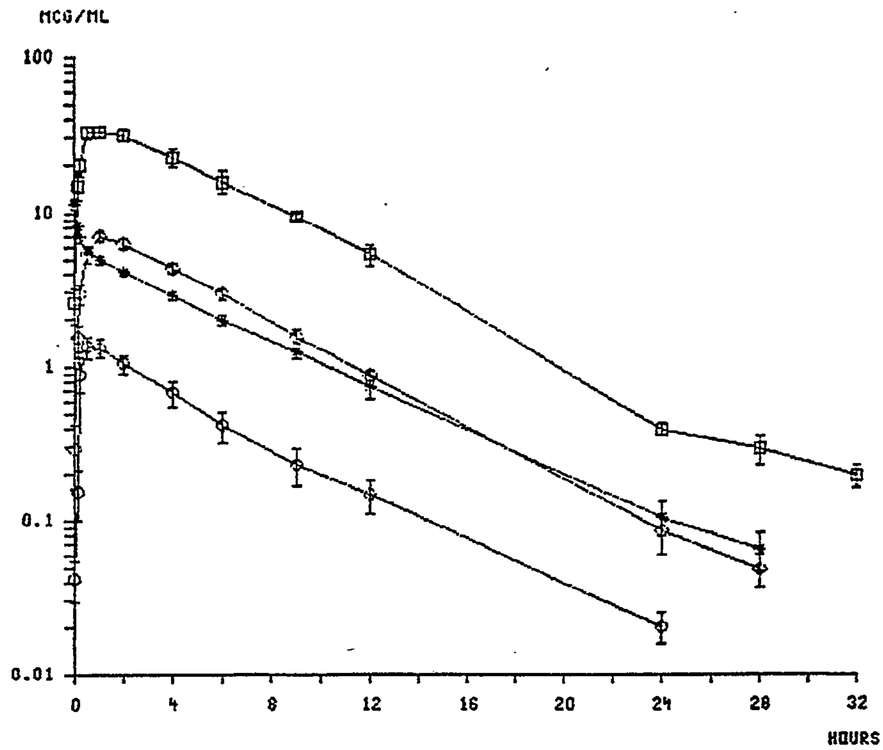


Abb. 3: Mittlerer Plasmaspiegel von 4-Acetamino-N-(2'-aminophenyl)-benzamid bei Ratten nach einmaliger Verabreichung von + 10 mg/kg i.v. (n = 5), o 2 mg/kg i.g. (n = 5), ◇ 10 mg/kg i.g. (n = 6), □ 50 mg/kg i.g. (n = 5). Halbwertzeit (t1/2) ~ 4,2 - 4,5 Stunden.

Die In-Vivo Aktivität von 4-Acetylamino-N-(2'-aminophenyl)-benzamid wurde getestet gegen das primäre Mammaadenokarzinom, induziert in weiblichen SD-Ratten durch drei einzelne i.V. Injektionen von Methylnitrosoharnstoff am 50., 71. und 92. Lebenstag. Sobald das Tumorvolumen ≥ 0,8 $cm^3$ erreichte, wurde nach Randomisierung der Versuchstiere die Therapie begonnen. Die Testsubstanz wurde intragastral in Dosen von 7,5 10,0 und 12,5 mg/kg/Tag 5 x /Woche in 5 Wochen verabreicht. Mittlere Tumorvolumen wurden wöchentlich bestimmt und mit unbehandelten Kontrolltieren verglichen.

Aus Abb. 4 ist ersichtlich, daß 4-Acetylamino-N-(2'-aminophenyl)-benzamid das Tumorwachstum deutlich beeinflußt in dosisabhängiger Weise bis zur vollständigen Verhinderung des Tumorwachstums in der Gruppe mit der höchsten Dosierung.

Abb. 4

Aktivität von 4-Acetamino-N-(2'-aminophenyl)-benzamid gegen Methylnitrosoharnstoff-induziertes Mamma-Adenokarzinom in SD-Ratten

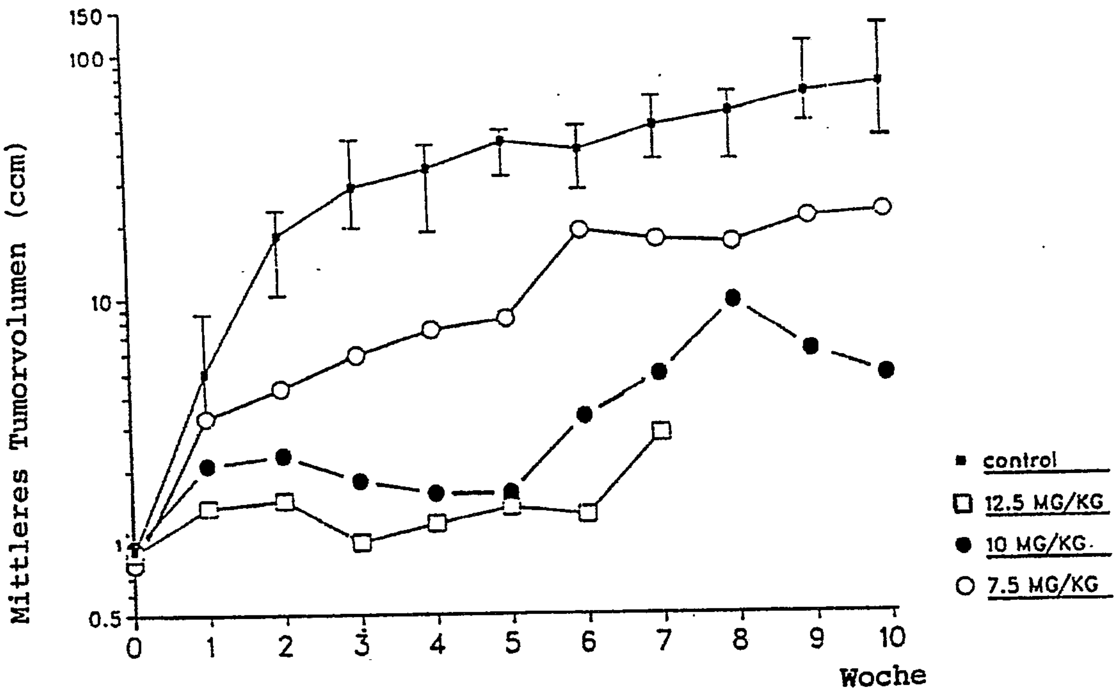


Die erfindungsgemäßen Substanzen stellen demnach gegenüber dem Vergleichsstandard 4-Amino-N-(2'-aminophenyl)-benzamid in wesentlichen biologischen Parametern deutlich verbesserte, dabei gegenüber L 1210 Leukämie-Zellen gleich wirksame und gegenüber Mammaadenokarzinom 16 C wirksamere Cytostatika dar.

Die erfindungsgemäßen Substanzen haben demnach therapeutische Bedeutung und stellen Mittel dar zur

- systemischen und/oder topischen Behandlung maligner Neoplasien
- Behandlung benigner proliferativer Erkrankungen
- Behandlung von Störungen des zellulären und humoralen Immunsystems.

Die Möglichkeit der Kombination mit Therapeutika, von denen zu erwarten ist, daß sie die gewünschten Effekte verstärken und günstig beeinflussen, ist mit eingeschlossen.

**Patentansprüche für die Vertragsstaaten: BE, CH, DE, FR, GB, IT, LI, LU, NL, SE**

1. N-2'-(Aminophenyl)-benzamid-Derivate der allgemeinen Formel II

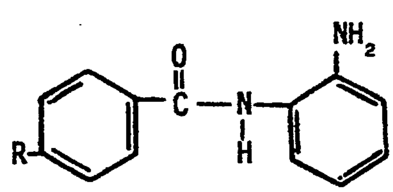

II

worin
R eine unsubstituierte oder mit Hydroxyl substituierte $C_1$–$C_4$-Acylaminogruppe bedeutet.

2. 4-Acetamino-N-(2'-aminophenyl)-benzamid.
3. 4-Isobutyrylamino-N-(2'-aminophenyl)-benzamid.
4. 4-Formylamino-N-(2'-aminophenyl)-benzamid.

5. 4-(β-Hydroxypropionyl)amino-N-(2'-aminophenyl)-benzamid.

6. Glycoloylamino-N-(2'-aminophenyl)-benzamid.

7. Verfahren zur Herstellung von Verbindungen gemäß Anspruch 1 dadurch gekennzeichnet, daß man Verbindungen der allgemeinen Formel III a und b:

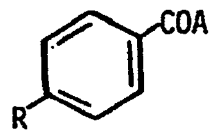
a)

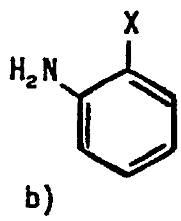
b)

(III a) + b)),

in welcher A eine reaktive Säuregruppe darstellt, R die obgenannte Bedeutung hat und X eine mit einer Schutzgruppe versehene Aminogruppe oder eine Nitrogruppe bedeutet,
in an sich bekannter Weise miteinander umsetzt, die so erhaltenen Verbindungen der allgemeinen Formel IV,

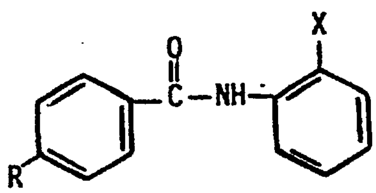
(IV),

in welcher X und R die obgenannte Bedeutung haben,
reduziert oder duch Abspaltung der Schutzgruppe in die entsprechenden Verbindungen der Formel II überführt.

8. Verwendung von Verbindungen der allgemeinen Formel II

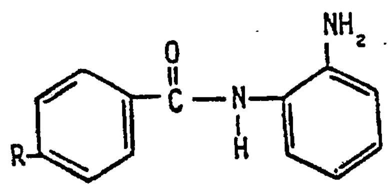
II

in welcher R Wasserstoff, eine Niederalkylalkoxy- oder eine Niederacylaminogruppe bedeutet, zur Herstellung eines pharmazeutischen Präparates für die Bekämpfung neoplastischer Erkrankungen.

9. Arzneimittel enthaltend Verbindungen gemäß Anspruch 8 neben Zusatz- und Hilfsstoffen.

**Patentansprüche für die Vertragsstaaten: AT, ES, GR**

1. Verfahren zur Herstellung von N-2'-(Aminophenyl)-benzamid-Derivaten der allgemeinen Formel II

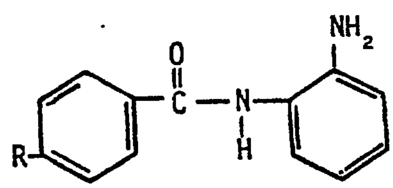
II

worin R eine unsubstituierte oder durch Hydroxyl substituierte $C_1$ bis $C_4$-Acylaminogruppe bedeutet, dadurch gekennzeichnet, daß man Verbindungen der allgemeinen Formel III a und b:

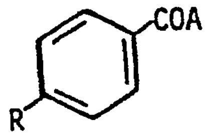 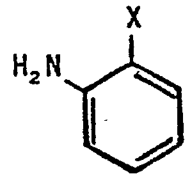 (III a) + b)),

a) b)

in welcher A eine reaktive Säuregruppe darstellt, R die obgenannte Bedeutung hat und X eine mit einer Schutzgruppe versehene Aminogruppe oder eine Nitrogruppe bedeutet,
in an sich bekannter Weise miteinander umsetzt, die so erhaltenen Verbindungen der allgemeinen Formel IV

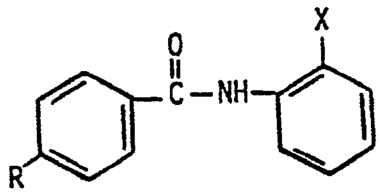 (IV),

in welcher X und R die obengenannte Bedeutung haben,
reduziert oder durch Abspaltung der Schutzgruppe in die entsprechenden Verbindungen der Formel II überführt.

2. Verfahren nach Anspruch 1 zur Herstellung von 4-Acetamino-N-(2′-aminophenyl)-benzamid.
3. Verfahren nach Anspruch 1 zur Herstellung von 4-Isobutyrylamino-N-(2′-aminophenyl)-benzamid.
4. Verfahren nach Anspruch 1 zur Herstellung von 4-Formylamino-N-(2′-aminophenyl)-benzamid.
5. Verfahren nach Anspruch 1 zur Herstellung von 4-(β-Hydroxypropionyl)amino-N-(2′-aminophenyl)-benzamid.
6. Verfahren nach Anspruch 1 zur Herstellung von Glycoloylamino-N-(2′-aminophenyl)-benzamid.
7. Verwendung von Verbindungen der allgemeinen Formel II

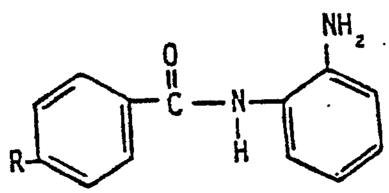 II

in welcher R Wasserstoff, eine Niederalkylalkoxy- oder eine Niederacylaminogruppe bedeutet, zur Herstellung eines pharmazeutischen Präparates für die Bekämpfung neoplastischer Erkrankungen.

**Claims for the Contracting States: BE, CH, DE, FR, GB, IT, LI, LU, NL, SE**

1. N-2′-(aminophenyl)-benzamide derivatives of the general formula II

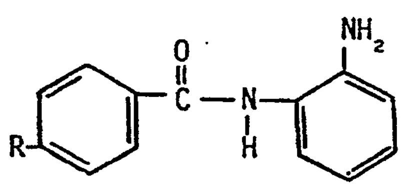 II

wherein R is an unsubstituted $C_1$–$C_4$ acylamino group or one substituted with hydroxyl.
2. 4-Acetamino-N-(2′-aminophenyl)-benzamide.
3. 4-Isobutyrylamino-N-(2′-aminophenyl)-benzamide.
4. 4-Formylamino-N-(2′-aminophenyl)-benzamide.
5. 4-(β-Hydroxypropionyl)amino-N-(2′-aminophenyl)-benzamide.
6. Glycoloylamino-N-(2′-aminophenyl)-benzamide.
7. Process for the preparation of compounds according to claim 1, characterised in that a compound of the general formula IIIa:

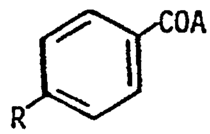

IIIa

a)

in which R has the same meaning as above and A is a reactive acid group, is reacted with a compound of the general formula IIIb

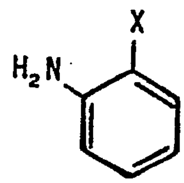

IIIb

b)

in which X is an amino group provided with a protective group or is a nitro group, in a manner known per se, the compounds thus obtained of the general formula IV

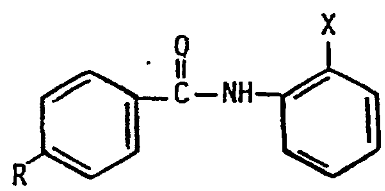

(IV),

in which X and R have the same meanings as above, are reduced or, by splitting off the protective group, are converted into the corresponding compounds of the general formula II.

8. Use of compounds of the general formula II

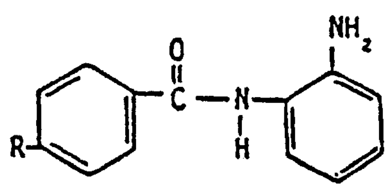

II

wherein R is hydrogen, a lower alkylalkoxy-or a lower acylamino group, for the production of a pharmaceutical preparation for combating neoplastic diseases.

9. Pharmaceutical compositions containing compounds according to claim 8 in addition to additional and auxiliary substances.

**Claims for the Contracting States: AT, ES, GR**

1. Process for the preparation of N-2'-(aminophenyl)-benzamide derivatives of the general formula II

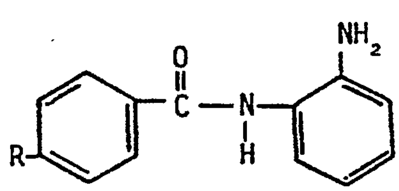

II

wherein R is an unsubstituted $C_1$–$C_4$ acylamino group or one substituted with hydroxyl, characterised in that a compound of the general formula IIIa

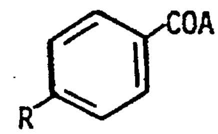

a)

IIIa

in which R has the same meaning as above and A is a reactive acid group, is reacted with a compound of the general formula IIIb

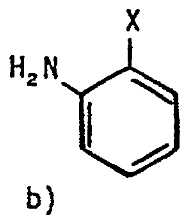

b)

IIIb

in which X is an amino group provided with a protective group or is a nitro group, in a manner known per se, the compounds thus obtained of the general formula IV

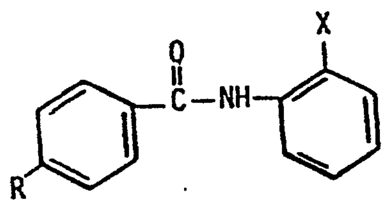

(IV),

in which X and R have the same meaning as above, are reduced or, by splitting off the protective group, are converted into the corresponding compounds of the general formula II.

2. Process according to claim 1 for the preparation of 4-Acetamino-N-(2′-aminophenyl)-benzamide.

3. Process according to claim 1 for the preparation of 4-Isobutyrylamino-N-(2′-aminophenyl)-benzamide.

4. Process according to claim 1 for the preparation of 4-Formylamino-N-(2′-aminophenyl)-benzamide.

5. Process according to claim 1 for the preparation of 4-(β-Hydroxypropionyl)amino-N-(2′-aminophenyl)-benzamide.

6. Process according to claim 1 for the preparation of Glycoloylamino-N-(2′-aminophenyl)-benzamide.

7. Use of compounds of the general formula II

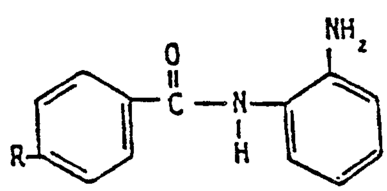

II

wherein R is hydrogen, a lower alkylalkoxy-or a lower acylamino group, for the production of a pharmaceutical preparation for combating neoplastic diseases.

**Revendications pour les Etats contractants: BE, CH, DE, FR, GB, IT, LI, LU, NL, SE**

1. Dérivés de N-2′-(aminophényl)-benzamide de formule générale II

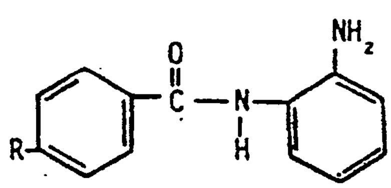

II

dans laquelle
R est un groupe acylamino en $C_1$–$C_4$ non-substitué ou substitué par un groupe hydroxyle.

2. 4-acétamino-N-(2′-aminophényl)-benzamide.
3. 4-isobutyrylamino-N-(2′-aminophényl)-benzamide.
4. 4-formylamino-N-(2′-aminophényl)-benzamide.
5. 4-(β-hydroxypropionyl)amino-N-(2′-aminophényl)-benzamide.
6. Glycoloylamino-N-(2′-aminophényl)-benzamide.
7. Procédé pour la préparation de composés selon la revendication 1, caractérisé en ce qu'on fait réagir d'une manière connue en soi, les uns avec les autres, des composés de formule générale IIIa et b

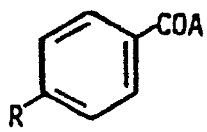
a)

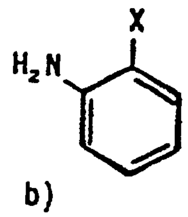
b)

(III a) + b)),

où A est un groupe acide réactif, R a les significations données ci-dessus et X est un groupe amino pourvu d'un groupe protecteur, ou un groupe nitro,
qu'on réduit les composés ainsi obtenus, de formule générale IV

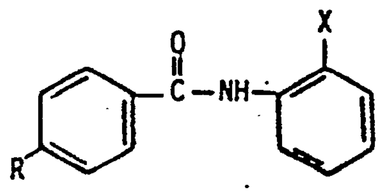
(IV),

dans laquelle X et R ont les significations données ci-dessus, ou qu'on les convertit en les composés correspondants de formule II par élimination du groupe protecteur.

8. Utilisation de composés de formule générale II

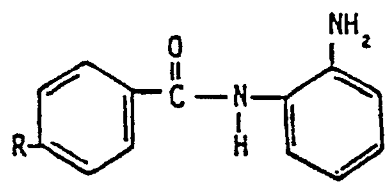
II

dans laquelle R est un hydrogène, un groupe alkylalcoxy inférieur ou acylamino inférieur,
pour obtenir une préparation pharmaceutique destinée à la lutte contre les maladies néoplasiques.

9. Médicament contenant outre les composés selon la revendication 8, des additifs et adjuvants.

**Revendications pour les Etats contractants: AT, ES, GR**

1. Procédé pour la préparation de dérivés de N-2′-(aminophényl)-benzamide de formule générale II

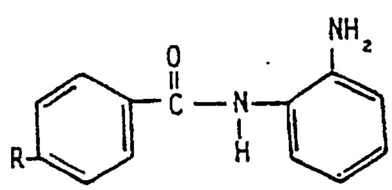
II

dans laquelle
R est un groupe acylamino en $C_1$–$C_4$ non-substitué ou substitué par un groupe hydroxyle,
caractérisé en ce qu'on fait réagir d'une manière connue en soi, les uns avec les autres, des composés de formule générale IIIa et b

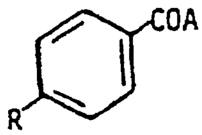

a)

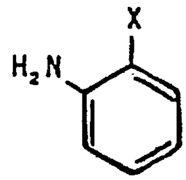

b)

(III a) + b)),

où A est un groupe acide réactif, R a les significations données ci-dessus et X est un groupe amino pourvu d'un groupe protecteur, ou un groupe nitro,
et qu'on réduit les composés ainsi obtenus, de formule générale IV

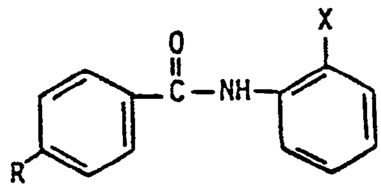

(IV),

dans laquelle X et R ont les significations données ci-dessus,
ou on les convertit en les composés correspondants de formule II par élimination du groupe protecteur.

2. Procédé selon la revendication 1, pour la préparation du 4-acétamino-N-(2'-aminophényl)-benzamide.

3. Procédé selon la revendication 1, pour la préparation du 4-isobutyrylamino-N-(2'-aminophényl)-benzamide.

4. Procédé selon la revendication 1, pour la préparation du 4-formylamino-N-(2'-aminophényl)-benzamide.

5. Procédé selon la revendication 1, pour la préparation du 4-(β-hydroxypropionyl)amino-N-(2'-aminophényl)-benzamide.

6. Procédé selon la revendication 1, pour la préparation du glycoloylamino-N-(2'-aminophényl)-benzamide.

7. Utilisation de composés de formule générale II

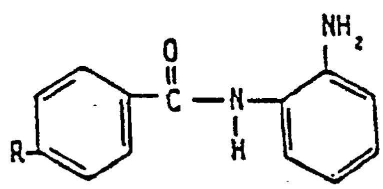

II

dans laquelle R est un hydrogène, un groupe alkylalcoxy inférieur ou acylamino inférieur,
pour l'obtention d'une préparation pharmaceutique destinée à la lutte contre les maladies néoplasiques.